(19) **Europäisches Patentamt**
**European Patent Office**
**Office européen des brevets**

(11) **EP 3 812 750 B1**

(12) **EUROPEAN PATENT SPECIFICATION**

(45) Date of publication and mention
of the grant of the patent:
**06.04.2022 Bulletin 2022/14**

(21) Application number: **20158248.3**

(22) Date of filing: **19.02.2020**

(51) International Patent Classification (IPC):
***G01N 22/02*** (2006.01)     ***G01N 33/38*** (2006.01)
***G01N 33/42*** (2006.01)     ***G01V 3/12*** (2006.01)

(52) Cooperative Patent Classification (CPC):
**G01N 33/42; G01N 22/02; G01N 33/383;**
**G01V 3/12**

(54) **SYSTEM AND METHOD FOR DETECTING POTHOLE SIGN OF ROAD PAVEMENT USING ELECTROMAGNETIC WAVE**

SYSTEM UND VERFAHREN ZUR DETEKTION EINES ANZEICHENS EINES SCHLAGLOCHES IN EINEM STRASSENBELAG UNTER VERWENDUNG ELEKTROMAGNETISCHER WELLEN

SYSTÈME ET PROCÉDÉ DE DÉTECTION DE SIGNE DE NID DE POULE DE REVÊTEMENT ROUTIER À L'AIDE D'ONDES ÉLECTROMAGNÉTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.10.2019 KR 20190130664**

(43) Date of publication of application:
**28.04.2021 Bulletin 2021/17**

(73) Proprietor: **Korea Expressway Corp.**
**Gyeongsangbuk-do, 39660 Republic of Korea (KR)**

(72) Inventors:
• **RHEE, Ji Young**
**13519 Seongnam-si, Gyeonggi-do (KR)**
• **SHIM, Jae Won**
**18429 Hwaseong-si, Gyeonggi-do (KR)**

• **KIM, Nag Young**
**13626 Seongnam-si, Gyeonggi-do (KR)**
• **KIM, Jin Hwan**
**18143 Osan-si, Gyeonggi-do (KR)**
• **KWON, Oh Sun**
**16910 Yongin-si, Gyeonggi-do (KR)**
• **KIM, Hyung Bae**
**13525 Seongnam-si, Gyeonggi-do (KR)**

(74) Representative: **ABG Intellectual Property Law, S.L.**
**Avenida de Burgos, 16D**
**Edificio Euromor**
**28036 Madrid (ES)**

(56) References cited:
**JP-A- 2010 014 472     KR-A- 20190 024 416**
**US-A1- 2019 094 202**

EP 3 812 750 B1

**Description**

BACKGROUND

The present invention relates to a method and system for detecting a sign associated with a pothole in a road pavement, and more particularly, to a method and system for providing information necessary for the maintenance decision of a road pavement by determining the crack rate increase of the road surface causing the pothole in the road pavement, the separation between the pavement layers, and the damages inside the pavement layer.

A pothole is a phenomenon in which as the surface of a pavement layer comes off, the road is pitted. Since the pothole can cause an accident of visitors using road immediately after occurrence, road managers have put a lot of effort into solving problems.

1) Quality and Construction Improvement Technology of Road Pavement materials: Korea Patent 10-1761165, 10-1635323, 2) Pothole real time detection and transmission technology: Korea Patent 10-1514368, 10-1546700, 3) Pothole prompt repair equipment and material development: Korean Patent 10-1675928, 10-1537247, 4) Pothole related accident compensation system.

1) is a technology related to improving the durability of road pavement. However, the damage associated with increased traffic volume, extreme weather (heavy rain, heavy snowfall, abnormal low temperatures, etc.), and the amount of snow-chloride spray further increased. Increasing the maintenance extension due to the expansion of the national road network and managing the limited maintenance budget caused the continued generation of potholes in the road pavement. In addition, because of the limitation of good quality aggregate materials and the use of low quality materials due to the cost burden, there are difficulties in maintaining the road pavement.

In the cases of 2), 3), and 4), the technique is related to the action after the pothole is generated. In the cases of 2) and 3), the pothole generated in the road pavement reduces the exposure time to the user, but it does not prevent the occurrence at the source. In the case of 4), it is related to the pothole complaint, and the evidence of the cause of the accident caused by the pothole is easily obtained so that related complaints and compensation work are on the rise. Due to this, administrative power and budget for road managers are wasted.

Document KR20190024416A discloses a device for evaluating a cavity formed under a road pavement, and more particularly, a buried pipe such as a water supply and sewage pipe or a gas pipe buried in a cavity generated by leakage of groundwater or water and sewage under the road pavement.

Document US2019/094202A1 discloses systems for asphalt density and soil moisture measurements using ground penetrating radar, and more particularly to a test instrument and method for measuring or correlating the density of an asphalt pavement sample using the reflection of high frequency signals from pavement material.

Document JP2010014472A discloses a non-destructive inspection device, a non-destructive inspection system, a non-destructive inspection vehicle, and the like, which are particularly preferably used for soundness diagnosis of highways and various general roads.

SUMMARY

The present invention provides a method and system for detecting a pothole sign of a road pavement, which can detect a pothole sign of a road pavement while minimizing errors, in detecting a stepwise sign that the pothole of a road pavement can occur.

In order to achieve the above object, a pothole sign detection system of a road pavement according to the present invention includes an electromagnetic wave transmitting unit, an electromagnetic wave receiving unit, a dielectric constant calculation unit, a crack rate diagnosis unit, a pavement layer separation state diagnosis unit, an attenuation calculation unit, a pavement layer inside state diagnosis unit, and a sign determination unit.

The pothole sign detection system of the present invention is defined in appended claim 1.

By such a configuration, non-destructive and simple detection is possible while minimizing errors for each stage state from the initial stage of the pothole that may occur in the road pavement to the occurrence, and since the pothole occurrence section of a road pavement can be easily identified and proactive maintenance is possible so that it contributes to ensuring the stability of passengers and roads.

At this time, a dielectric constant of the road pavement surface is calculated by Equation:

$$\sqrt{\epsilon_{r0}} = \frac{1 + Amp_0}{1 - \dfrac{Amp_0}{Amp_P}} \,,$$

where $\epsilon_{r0}$ is a dielectric constant of the road surface, $Amp_0$ is an amplitude of the electromagnetic wave at the road surface, and $Amp_P$ is an amplitude of the electromagnetic wave at a steel plate (complete reflection).

In addition, a dielectric constant of the pavement layer interface is calculated by Equation:

$$\sqrt{\epsilon_{rn}} = \sqrt{\epsilon_{r(n-1)}} \times \left[ \frac{1 - \left(\frac{Amp_{(n-1)}}{Amp_p}\right)^2 + \left(\frac{Amp_n}{Amp_p}\right)}{1 - \left(\frac{Amp_{(n-1)}}{Amp_p}\right)^2 - \left(\frac{Amp_n}{Amp_p}\right)} \right],$$

where $\epsilon_{rn}$ is a dielectric constant of the n-th pavement interface of the pavement road, and $Amp_n$ is an amplitude of the electromagnetic wave at the n-th pavement interface of the road pavement.

In addition, the attenuation is calculated by Equation:

$$\delta_n(dB) = 20\log_{10}\frac{Amp_n}{Amp_p} - 20\log_{10}\frac{Amp_{(n-1)}}{Amp_p},$$

where $\delta_n$ is an attenuation (dB) of the electromagnetic wave passing through the n-th pavement layer of the road pavement.

In addition, the pothole sign detection system may further include a reference value correction unit for correcting a surface dielectric constant reference value and an interface dielectric constant reference value according to an environment in a road pavement. By such a configuration, more accurate detection of pothole sign is possible by reflecting various environmental factors that can change the dielectric constant of a road pavement measured by electromagnetic wave.

At this time, the environment is age and relative humidity,

wherein the dielectric constant reference value is calculated by Equation $\epsilon_r = A \cdot W_r - B\ln(Age) + C$, where $\epsilon_r$ is the dielectric constant reference value, $W_r$ is a water content or relative humidity (%), Age is the material age of concrete or public use(years), and A, B, C are predetermined constants.

Moreover, when the pavement layer is asphalt, B is set to 0, and A and C are differently set for the surface of the road pavement and the internal pavement interface, wherein A is set to be smaller by a predetermined ratio as compared to the surface of the road pavement for the internal pavement interface of the road pavement.

Furthermore, when the pavement layer is concrete, A, B, and C are differently set for the surface of the road pavement and the internal pavement interface, respectively, wherein B is set to be smaller than the surface of the road pavement for the internal pavement interface of the road pavement.

In addition, the environment is a density of a pavement layer of the road pavement, wherein the dielectric constant reference value is calculated by Equation $\epsilon = a\rho + b$, where $\epsilon$ is the dielectric constant reference value, $\rho$ is the density of a core specimen of the road pavement, and a and b are constants.

In addition, the reference value correction unit may further correct the attenuation reference value according to the environment in the road pavement. By such a configuration, more accurate detection of pothole sign is possible by reflecting various environmental factors that can change the attenuation of electromagnetic wave reflected from a road pavement.

At this time, the attenuation reference value is calculated by Equation $\sigma = a\rho - b$, where $\sigma$ is the attenuation reference value, $\rho$ is the density of a core specimen of the road pavement, and a and b are constants.

Moreover, disclosed is a pothole sign detection method, performed by the pothole sign detection system, as defined in appended claim 12.

BRIEF DESCRIPTION OF THE FIGURES

The accompanying drawings are included to provide a further understanding of the inventive concept, and are incorporated in and constitute a part of this specification. The drawings illustrate exemplary embodiments of the inventive concept and, together with the description, serve to explain principles of the inventive concept. In the drawings:

FIG. 1 is a schematic block diagram of a pothole sign detection system of a road pavement in accordance with the present invention;

FIG. 2 is a schematic block diagram illustrating one embodiment of FIG. 1;

FIG. 3 is a graph of an example electromagnetic wave reception from a road pavement;

FIG. 4 is a graph showing an example of the relationship between the pavement density and the dielectric constant of a road pavement;

FIG. 5 is a graph showing an example of the relationship between a dielectric constant and a distribution ratio;

FIG. 6 is a graph comparing the pavement density and attenuation in the actual road pavement; and

FIG. 7 is a schematic flowchart of a pothole sign detection method according to an embodiment of the present invention.

## DETAILED DESCRIPTION

Hereinafter, preferred embodiments of the present invention will be described with reference to the accompanying drawings.

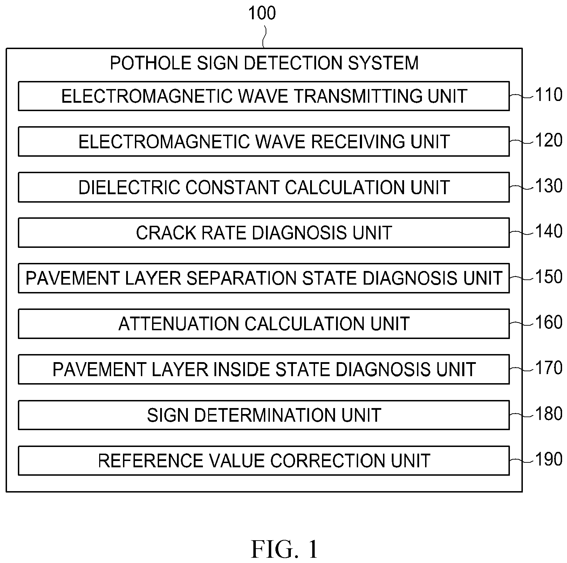

FIG. 1 is a schematic block diagram of a pothole sign detection system of a road pavement in accordance with the present invention. In FIG. 1, a pothole sign detection system 100 includes an electromagnetic wave transmitting unit 110, an electromagnetic wave receiving unit 120, a dielectric constant calculation unit 130, a crack rate diagnosis unit 140, a pavement layer separation state diagnosis unit 150, an attenuation calculation unit 160, a pavement layer inside state diagnosis unit 170, a sign determination unit 180, and a reference value correction unit 190.

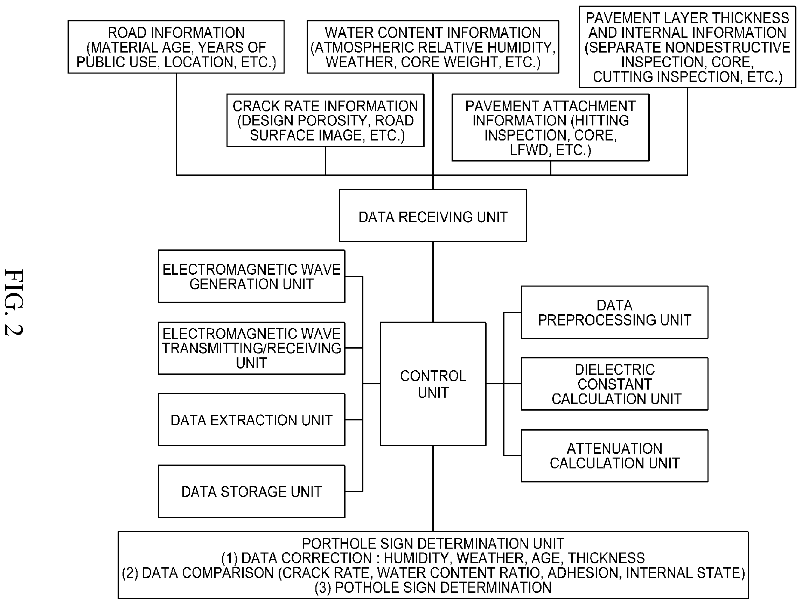

FIG. 2 is a schematic block diagram illustrating one embodiment of FIG. 1. In FIG. 2, an example of a pothole sign detection system of a road pavement is shown that includes an electromagnetic wave generation unit for generating electromagnetic wave, an electromagnetic wave transmitting/receiving unit for emitting electromagnetic waves generated from the electromagnetic wave generation unit to road pavements and receiving electromagnetic waves reflected from the interface of each pavement layer, a dielectric constant calculation unit for analyzing the electromagnetic wave received from the electromagnetic wave receiving unit and calculating the dielectric constant of each pavement layer by the amplitude of the received wave reflected from each layer of the road pavement, an attenuation calculation unit for calculating the attenuation of the electromagnetic wave energy passing through the pavement layer by the amplitude difference in each layer, and a pothole sign determination unit for detecting a pothole sign of a road pavement from a calculated dielectric constant and attenuation.

The occurrence mechanism of potholes is mainly known for 1) poor compaction during construction of pavement materials, so that harmful substances penetrate at places with large porosity and 2) as the road material separated from the internal moisture escapes during vehicle weight load, bearing capacity is weakened, so that depressions and potholes occur.

In addition, as a result of the recent on-site survey by the Korea Expressway Corporation, the following additional causes were found. 1) The penetration path of harmful substances increases due to the increase of the overall crack rate due to the increased crack rate of the road pavement in public use (longitudinal and transverse cracks, craze cracks, aggregate separation, construction joints, etc.), 2) Pothole occurs because the upper layer is dropped due to poor adhesion between the upper and lower layers of the pavement, 3) Potholes occur due to the deterioration and dropping of the upper layer resulting from the weakening of bearing capacity due to internal damage of the pavement layer (layer separation, lower layer damage, etc.), and 4) Pothole occurs because the upper layer is dropped due to poor waterproofing of an upper and lower layer interface part.

In the event of a site survey, if a pothole related damage occurs on the road surface, there were many cases (66%) where related damage occurred internally but if the road surface was good, there were cases (30%) in which damage occurred inside. This can usually be found in one place, such as re-pavement or cutting overlay.

In other words, if the road surface is good but there is a wide range of damage inside, in a case where there is no fundamental repair, increasing damage will likely burden the road management agency in the long run. Currently, many organizations detect damages by road surface images, but there are technical limitations for these reasons. Therefore, the inventors devised a method of exploring the surface from the inside using electromagnetic waves.

The main causes of damage to potholes can be categorized into 1) increased crack rate on the road surface, 2) separation between pavement layers, and 3) internal damage to the pavement layer.

In relation to electromagnetic waves, the reception signal changes depending on the material properties and density of the medium that transmits electromagnetic waves, and whether damage occurs. In other words, a change occurs in the amplitude and energy loss of the received wave and this can be represented by dielectric constant and attenuation, which are electromagnetic wave characteristic values.

The dielectric constant represents the material properties and density of the medium and generally is in the range of air 1, water 81, asphalt 3 to 5, and concrete 5 to 10. By comparing the electromagnetic wave reception amplitude on the road with the amplitude on the steel plate in the field, calculation is made by the following equation.

Equation 1 :

$$\sqrt{\epsilon_{r0}} = \frac{1 + Amp_0}{1 - \dfrac{Amp_0}{Amp_P}}$$

Equation 2 :

$$\sqrt{\epsilon_{rn}} = \sqrt{\epsilon_{r(n-1)}} \times \left[ \frac{1 - \left(\dfrac{Amp_{(n-1)}}{Amp_p}\right)^2 + \left(\dfrac{Amp_n}{Amp_p}\right)}{1 - \left(\dfrac{Amp_{(n-1)}}{Amp_p}\right)^2 - \left(\dfrac{Amp_n}{Amp_p}\right)} \right]$$

where $\epsilon_{r0}$ is a dielectric constant of the road surface, $\epsilon_{rn}$ is a dielectric constant of the n-th pavement interface, $Amp_0$ is an amplitude at the road surface, $Amp_n$ is an amplitude at the nth pavement interface, and $Amp_P$ is an amplitude at the steel plate (complete reflection).

Attenuation refers to the energy loss of electromagnetic waves propagating through a medium, and is generally caused by the thickness, electrical conductivity, and scattering of the medium. The attenuation generated in the pavement layer can be calculated by the difference between the amplitudes of the upper and lower surfaces of the pavement layer. The unit uses gain (dB).

Equation 3 :

$$\delta_n (dB) = 20\log_{10} \frac{Amp_n}{Amp_p} - 20\log_{10} \frac{Amp_{(n-1)}}{Amp_p}$$

where $\delta_n$ is the attenuation (dB) of the electromagnetic wave passing through the n-th layer

The electromagnetic characteristic values of the electromagnetic wave are summarized in relation to the main damage cause of the pothole as follows. 1) Crack rate is increased -> pavement layer upper surface dielectric constant dry condition becomes smaller and water content condition becomes larger, 2) Separation between pavement layers -> pavement layer interface dielectric constant dry condition becomes smaller and water content condition becomes larger, and 3) Pavement layer internal damage -> pavement layer attenuation drying condition becomes slightly smaller and water content condition becomes slightly larger.

In addition, the threshold value (range) of asphalt and concrete, which are main media constituting the road pavement, may be used by Korea Highway Corporation.

1) Dielectric constant

Asphalt dielectric constant threshold value (range): 4.5 to 6.5

Concrete dielectric constant threshold value (range): change value according to material age (or period of public use)

Equation 4 : $[-A \times \ln(Age) + C] \pm error$

where A and C are constants, Age is a concrete material age (year), and error is a fluctuating range. A may be 1.08, C may be 11.41, and Age uses bridge information and can be replaced by years of public use.

2) Attenuation

Asphalt attenuation threshold value : 1.26dB or less per 10mm

Concrete attenuation threshold value : 1.95dB or less per 10mm

With this technical background, a pothole sign prediction method using an electromagnetic wave by the pothole sign detection system 100 of FIG. 1 will be described with reference to the drawings.

1) Crack rate evaluation

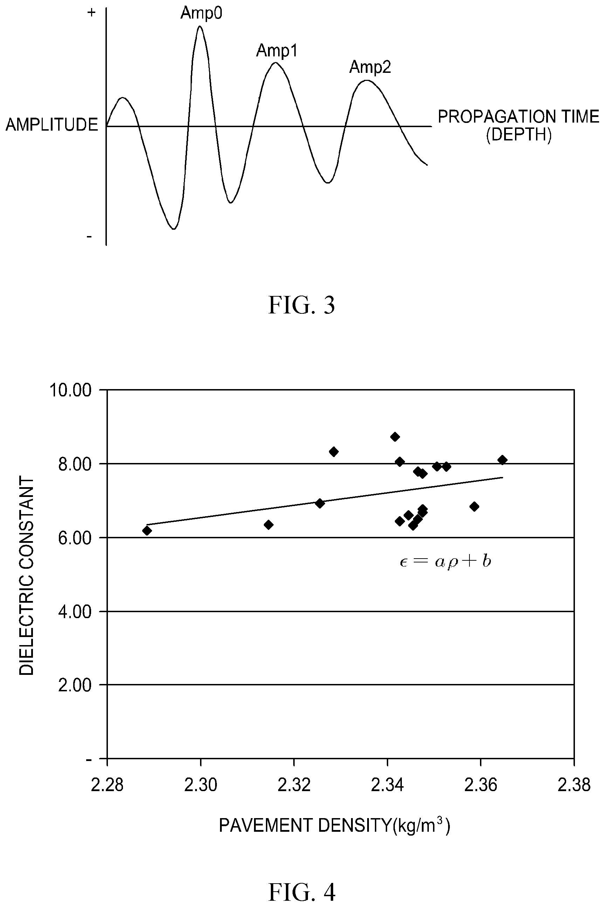

The electromagnetic wave transmitting unit 110 emits electromagnetic waves, and the electromagnetic wave receiving unit 120 extracts amplitudes from the road pavement surface and each layer. FIG. 3 is a graph of an example electromagnetic wave reception from a road pavement.

The dielectric constant calculation unit 130 obtains the dielectric constant using Equations 1, 2, and 4, and the reference value correction unit 190 uses the road surface image and the water content rate information to correct the dielectric constant threshold value. (e.g., compare and adjust the dielectric constant threshold value (range) in comparison with crack generation part or water content part in road surface image, visual inspection, appearance network, etc.)

The crack rate diagnosis unit 140 compares the calculated dielectric constant with the corrected threshold value, and the sign determination unit 180 classifies as the pothole generation region when the calculated dielectric constant is out of the threshold value (range).

2) Evaluation of adhesion between pavement layers

The electromagnetic wave transmitting unit 110 emits a received electromagnetic wave, and the electromagnetic wave receiving unit 120 extracts the amplitude from the road pavement surface and each layer. The dielectric constant calculation unit 130 obtains the dielectric constant using Equations 2 and 4, and the reference value correction unit 190 corrects the dielectric constant threshold value (range) using the pavement layer attachment information. (e.g., compare pavement layer separation sections such as beating inspection, core, LFWD, etc. to compare and adjust the dielectric constant threshold value between the pavement layers.)

The pavement layer separation state diagnosis unit 150 compares the calculated dielectric constant with the corrected threshold value, and the sign determination unit 180 classifies as a pothole occurrence or a sign region when the calculated dielectric constant is out of the threshold value (range).

3) Pavement layer inside evaluation

The electromagnetic wave transmitting unit 110 emits electromagnetic waves, and the electromagnetic wave receiving unit 120 extracts amplitudes from the road pavement surface and each layer. The attenuation calculation unit 160 calculates attenuation in each pavement layer using Equation 3, and the reference value correction unit 190 corrects an attenuation threshold value (range) by using internal information of the pavement layer. (e.g., compare the damage area inside the pavement layer such as core, design thickness, cutting inspection, etc. to compare and adjust the threshold value (range) of the pavement layer attenuation.)

The pavement layer inside state diagnosis unit 170 compares the calculated attenuation with the corrected threshold value, and the sign determination unit 180 classifies as the pothole occurrence or the sign region when the calculated attenuation exceeds the threshold value.

As described above, the reference value correction unit 190 corrects the reference value using various factors. First, the reference value correction of the dielectric constant by age and relative humidity is described as follows.

According to a recent study, the Korea Highway Corporation has found that the main factor affecting the dielectric constant of the pavement layer is the water content ratio (relative humidity when exposed to air) and the material age also affects greatly in the case of concrete pavement. (Existing dual log relationship, improvement log relationship)

The influence equation of a water content ratio and an material age for the dielectric constant can be expressed as Equation 5.

$$\text{Equation 5:} \quad \epsilon_r = A \cdot W_r - B \ln(Age) + C$$

where $\epsilon_r$: a dielectric constant of a pavement layer, $W_r$: a water content ratio or relative humidity (%), Age: concrete material age or years of public use(years), A, B, C: constants

Korea Expressway Corporation's empirical formula for Equation 5 is as follows. The dielectric constant change rate of the N-th pavement layer according to the water content ratio (or relative humidity) is about 1/8 of the dielectric constant change rate of the first pavement body exposed to air (Equations 6 and 7, 1 GHz frequency example). In other words, since the internal pavement layer, compared to the dielectric constant change rate of the first pavement body, which is exposed to air and whose water content ratio varies with the circulation of the atmosphere, the change rate will

appear small.

$$\text{Equation 6:}\quad \epsilon_{r,N}^{RH} = 0.01\,RH + 8.09$$

$$\text{Equation 7:}\quad \epsilon_{r,1st}^{RH} = 0.08\,RH + 4.34$$

where $\epsilon_{r,N}$ is a dielectric constant of an internal pavement layer, $\epsilon_{r,1st}$ is a dielectric constant of the first pavement layer, and RH is a relative humidity of the atmosphere (%)

In this case, the influence of a relative humidity can be ignored at a relative humidity of 70% or less.

In the case of a concrete pavement body, it is more affected by material age than relative humidity, and the dielectric constant change rate of the N-th pavement layer is less than the dielectric constant of the first pavement body exposed to air. (Equations 8 and 9, 1 GHz frequency example) The first pavement body is in contact with the dry atmosphere so that while the excess water used for concrete mixing evaporates and hardens quickly, the internal concrete pavement layer has a relatively low rate of decrease in dielectric constant due to blocking of outside air.

$$\text{Equation 8:}\quad \epsilon_{r,N}^{Age} = -\,1.08\ln(Age) + 11.41$$

$$\text{Equation 9:}\quad \epsilon_{r,1st}^{Age} = -\,1.63\ln(Age) + 12.17$$

where Age is the material age of concrete or the period of public use (years)

At this time, it can be ignored for more than 25 years of public use.

Next, the dielectric constant reference value correction by measuring the design density or the porosity of the core specimen is as follows. The relation between the dielectric constant and the density of a pavement layer in public use is as follows. In other words, as the density is larger, the dielectric constant becomes larger.

Since density correlates with compaction, which is the main factor of potholes, through the density analysis of the core, possible pothole areas can be found by using the correlation with the dielectric constant.

$$\text{Equation 10:}\quad \epsilon = a\rho + b$$

where $\epsilon$: a dielectric constant of a pavement body, $\rho$: a core density (kg/m3), a and b are constants determined by the density test of the core. FIG. 4 is a graph illustrating an example of a relationship between a pavement density and a dielectric constant of a road pavement.

In this case, the dielectric constant reference value may be set to a specific threshold value, but may be set to a threshold range of a predetermined range. More specifically, when the state of the pavement layer is good, the distribution of the dielectric constant has high kurtosis and low dispersion.

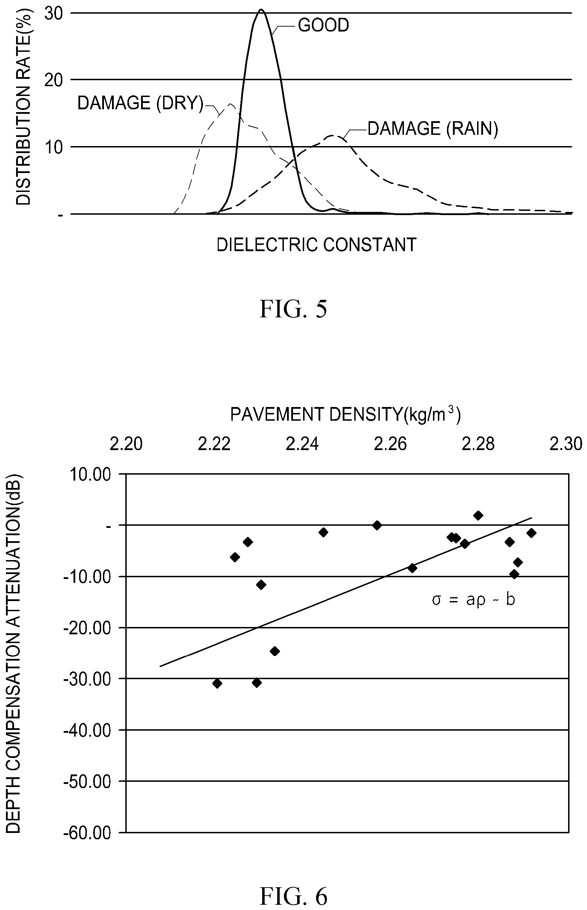

But if damage occurs, the kurtosis is low, the center value is large in water content conditions, and the dispersion is large. In the dry condition, the central value is small and the dispersion is relatively small compared to the water content condition. FIG. 5 is a graph illustrating an example of a relationship between a dielectric constant and a distribution ratio.

Therefore, the reference value of the dielectric constant can be determined by the formula, but it is desirable for maintenance agencies to use coefficients of variation to determine critical ranges. The coefficient of variation may vary depending on the equipment but at 85% confidence, a range of 0.2 to 0.25 can be selectively used depending on the level of maintenance. The soundness criterion is shown in Equation 11. The dielectric constant of the pavement layer can be classified as healthy if it is within the upper and lower limits, respectively and otherwise, can be classified as damaged.

$$\text{Equation 11:}\quad (1 - 1.5\,CV)\epsilon_r < \epsilon_{r,survey} < (1 + 1.5\,CV)\epsilon_r$$

where CV is the coefficient of variation (0.2 to 0.25), $\epsilon_r$: a dielectric constant reference value of a pavement, and $\epsilon_{r,\text{survey}}$: a dielectric constant of a pavement layer measured in the field

Other considerations are as follows. One of the pothole factors is road surface damage such as cracking. In other words, it is possible to determine the dielectric constant reference value by the appearance network or road surface image. The methods are as follows: 1) dielectric constant measurement, 2) setting a dielectric constant threshold range consistent with road surface damage, and 3) sound within critical range, and otherwise, damage.

In addition, the pothole factor in the N-th pavement layer is the lack of adhesion or separation between pavement layers, and the separated section can be measured by hitting sound. If low pitched sound occurs by hitting the upper pavement layer (pounding inspection, LFWD11 Light Falling Weight Deflectometer), it can be determined that there is a lifting part inside.

Therefore, corrections such as 1) dielectric constant measurement, 2) setting of the dielectric constant threshold range consistent with the lifting part, and 3) sound within critical range, and otherwise, damage are possible.

In addition, it was confirmed in actual field survey that different values may appear depending on the frequency of the electromagnetic wave or by user manipulation such as hardware, software, and filtering of the equipment. Therefore, it is very important to obtain data that is close to the actual one through various corrections.

Meanwhile, the reference value correction for the attenuation of the reference value correction unit 190 may also be performed. The total attenuation generated from the electromagnetic wave passing through the pavement body can be expressed as in Equation 12.

$$\text{Equation 12: } \delta_{layer} = \delta_{depth} + \delta_{damage}$$

where $\delta_{layer}$ is a total attenuation under a pavement layer, $\delta_{depth}$ is an attenuation due to a change of the thickness of a pavement layer, and $\delta_{damage}$ is an attenuation due to the conductivity or damage (aggregate separation, etc.) of a pavement body

When description is made through an example of correction for porosity, a place where the conductivity of the pavement body is high is a place where the porosity is large so that rainwater and snow removal chloride are excessively infiltrated. Therefore, a place where an attenuation of a pavement body occurs greatly is a place where porosity is large, that is, density is small.

FIG. 6 is a graph comparing density and attenuation in an actual pavement body in public use. Depth correction attenuation without the effect of depth has a correlation with the crack rate, so this quality assessment can be performed through this relationship.

$$\text{Equation 13: } \sigma = a\rho - b$$

where $\sigma$: a depth correction attenuation (dB) at a pavement body lower surface, $\rho$: a core density (kg/m3), and a and b are constants determined by a core density test

At this time, the critical range of depth correction attenuation is generally defined as 6 to 8dB level, and it is common to determine the reference value through the core and repairing inspection (a task of examining a pavement layer and an internal damage while removing a pavement layer).

However, this value becomes smaller as a maintenance level of a manager is higher, and becomes larger as the frequency of the electromagnetic wave is greater. In addition, it can be larger when inspected with high water content ratios. Therefore, when setting the threshold value of the attenuation, a task for correction is necessary through inspection of core, and the like.

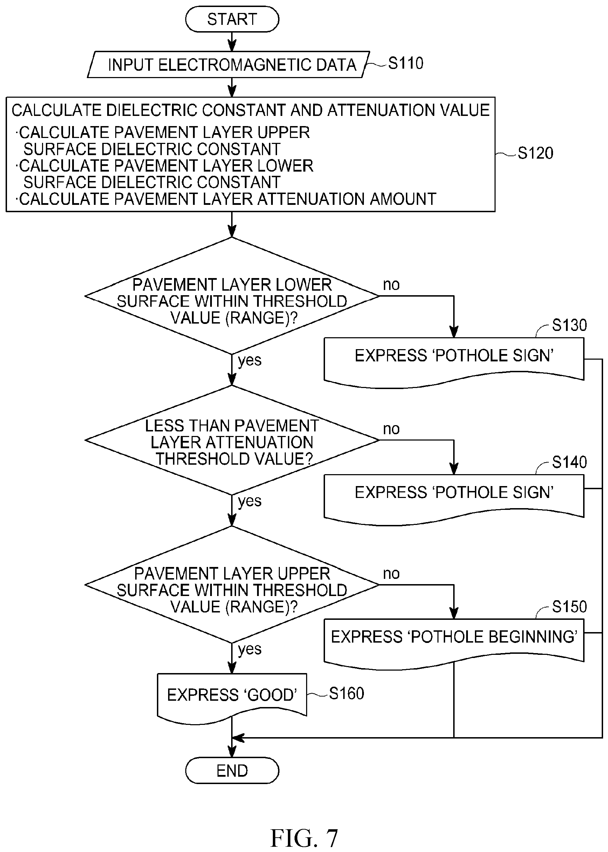

FIG. 7 is a schematic flowchart of a pothole sign detection method according to an embodiment of the present invention. FIG. 7 shows an example of a pothole evaluation algorithm by electromagnetic waves.

First, an electromagnetic wave is generated, the generated electromagnetic wave is emitted to a road pavement, and an electromagnetic wave reflected from an interface of each pavement layer is received (S110). Subsequently, the dielectric constant of the road pavement is calculated based on the amplitude of the received electromagnetic wave, and the attenuation of energy is calculated using the amplitude difference of the received electromagnetic wave in each layer of the road pavement (S120).

Next, through a comparison between the calculated interface dielectric constant of the N-th and (N-1)-th road pavement layers and the dielectric constant threshold value (range), the adhesion and state between road pavement layers are evaluated in order to evaluate the pothole sign of the road pavement, and when it is evaluated as a pothole sign, it is expressed (S130).

More specifically, the dielectric constant is out of the threshold value, it is evaluated as the pothole generating part due to the decrease in adhesion between the pavement layers, and if the threshold value (range) is below the dielectric constant, it is evaluated as lifting (drying condition) and if not, it is evaluated as a water content condition state.

Next, through a comparison between the calculated attenuation and the attenuation amplitude threshold value, the pothole sign of the road pavement is evaluated by evaluating the state in each layer of the road pavement, and when it is evaluated as a pothole sign, it is expressed (S140).

More specifically, when it is below a threshold value of attenuation, it is evaluated as a pothole generating part due to internal damage, and it can be evaluated that internal damage is due to layer separation, aggregate separation, or harmful substance penetration state.

The threshold value of the attenuation can be applied considering the error range to 1.26dB per 10mm depth of asphalt pavement and 1.95dB per 10mm depth of concrete pavement (experience value). At this time, the threshold value of the attenuation may vary depending on the type of mixture of the road pavement (density, SMA, drainage pavement, fiber mixed concrete, etc.), the frequency and equipment characteristics of the electromagnetic wave, and the maintenance level of the management agency.

Next, through a comparison between the dielectric constant of the estimated road pavement surface and the dielectric constant threshold value (range), the pothole sign is evaluated by evaluating the state of the road surface, and when it is evaluated as a pothole sign, the pothole initial signal is expressed (S150).

More specifically, when the dielectric constant is out of the threshold value (range), the crack hole (or porosity) excess is evaluated as a pothole possible part. In this case, the crack rate is an index converted into the degree of tightness of the road surface, that is, the penetration of harmful substances and is increased by damage such as porosity (compaction failure), cracks, construction joints, aggregate separation, and the like.

The range below the threshold value of the dielectric constant is evaluated as an excessive void (dry condition), and the range above it is evaluated as a water content condition state. In the case of a new construction, it is evaluated as mixture or poor compaction, and in the case of maintenance, it is evaluated as the development of related damage (increase in crack rate such as cracks, construction joints, potholes, etc.).

In relation to the threshold value of the dielectric constant, asphalt road pavement can be set at 4.5 to 6.5 and concrete road pavement can be set at $[-A \times \ln (Age) + C] \pm error$ (A and C are constants, Age is a concrete age (year), error is a fluctuating range, and as an experience value, A can be replaced by 1.08, C can be replaced by 11.41, and Age can be replaced by years of public use) (experience value).

At this time, the threshold value (range) of the dielectric constant may vary depending on the type of a mixture of a road pavement (density, SMA, drainage pavement, fiber mixed concrete, etc.), frequency, equipment characteristics, and maintenance levels.

Finally, if it is not evaluated as the sign of a pothole in the above process, a good signal is expressed (S160).

The evaluation criteria for the pothole sign and their meanings are summarized as follows.

[Table 1]

| Detection target | Road surface crack rate | Adhesive between pavement layers | Whether there is damage in pavement layer | Remarks |
|---|---|---|---|---|
| Classification factor | Road surface dielectric constant | Interface dielectric constant between pavement layers | pavement layer attenuation | |
| Calculation method | Equation 1 | Equation 2 | Equation 3 | |
| Threshold value(range) | Asphalt 4.5~6.5 Concrete [- $A \times \ln (Age) + C] \pm error$ | Same as left | Asphalt attenuation 1.26dB or less per 10 mm, Concrete attenuation 1.95dB or less per 10 mm | Empirical formula |
| State evaluation | Within threshold value(range): road surface is good, Other than threshold value (range): crack rate increase (New compaction defect, increased crack rate) | Within threshold value (range): Adhesive between pavement layers is good, Other than threshold value (range): pavement layer separation (threshold value (range) of less lifting or cavity occurrence, staying water more than threshold value (range)) | Within threshold value (range): pavement layer inside is good, Other than threshold value (range): pavement layer inside layer separation, aggregate separation, snow removal chloride accumulation | |

(continued)

| Calculation method | Equation 1 | Equation 2 | Equation 3 | |
| --- | --- | --- | --- | --- |
| Pothole | Early stage, attention observation | Pothole occurrence possibility due to dropping of interface between pavement layers | Pothole occurrence possibility due to dropping of pavement layer or weakening bearing capacity | |

Each of determination steps 130, 140, and 150 of the pothole sign can be used independently or in combination, respectively, and when using a combination of two or three steps, the pothole sign can be better evaluated as follows.

1) Road surface and interface dielectric constant are out of threshold value (range), and the pavement layer attenuation is below the threshold value: due to poor compaction or crack rate in an overall pavement layer, harmful substances penetration is possible inside the pavement body and the interface between pavement layers is separated so that forming a pothole generating condition of the upper layer, and it is highly likely to occur in places where construction is poor (adhesive surface treatment and compaction failure) during new or re-pavement.

2) The dielectric constant of the road surface is out of threshold value (range), the interface dielectric constant range between pavement layers is within the threshold range, and pavement layer attenuation threshold value exceeds the threshold value: adhesion between pavement layers is good, but due to the increase in the crack rate of the surface of the pavement layer, harmful substances penetrate and damage occurs inside so that forming a pothole generating condition on the upper part of the pavement layer. In heavy vehicles or heavy traffic, it is highly likely to occur when snow removal chloride accumulates inside through crack damage on road surface

3) The road surface dielectric constant is within the threshold value (range), the interface dielectric constant between pavement layers is within the threshold value, and the pavement layer attenuation is below the threshold value: The state of the road surface seems to be good due to sealing, but poor adhesion between pavement layers and damage inside pavement layer occur, and thus bearing capacity is weakened, and when the vehicle passes by, the upper layer is recessed so that the pothole generation condition of the damaged part can be continuously formed. It is likely to occur in one place where there is a cutting overlay and asphalt is overlaid on aging concrete pavement

4) The road surface and the interface dielectric constant is out of threshold values(range) and the pavement layer attenuation exceeds threshold values: As the crack rate increases in the road surface, harmful substances penetrate, the pavement layers are separated, and internal damage occurs. Potholes can occur constantly. It is likely to occur in old age pavement

In short, the present invention relates to a method and system for detecting a sign associated with a pothole and predicting a step of forming the pothole using an electromagnetic wave in a road paving layer. More specifically, the present invention relates to a method and system for immediately providing information necessary for maintenance decision of a road pavement by calculating a dielectric constant and attenuation of pavement material, comparing it to the threshold value, and determine the formation conditions and stages of pothole using the crack rate increase of the surface of the road to induce potholes in new and public road pavements, separation between pavement layers, and the damage inside the pavement layer through the received signal (amplitude of each layer) of the electromagnetic wave.

In the present invention, the electromagnetic wave is used to detect damage signs associated with potholes generated in the road pavement and immediately transmit them to the management authority. The present invention provides a pothole sign detection method and system of a road pavement that includes an electromagnetic wave generation unit for generating electromagnetic wave, an electromagnetic wave transmitting/receiving unit for emitting electromagnetic waves generated from the electromagnetic wave generation unit to road pavements and receiving electromagnetic waves reflected from the interface of each pavement layer, a dielectric constant calculation unit for analyzing the electromagnetic wave received from the electromagnetic wave receiving unit and calculating the dielectric constant of each pavement layer by the amplitude of the received wave reflected from each layer of the road pavement, an attenuation calculation unit for calculating the attenuation of the electromagnetic wave energy passing through the pavement layer by the amplitude difference in each layer, and a pothole sign determination unit for detecting a pothole sign of a road pavement from an estimated dielectric constant and attenuation.

According to the present invention, non-destructive and simple detection is possible while minimizing errors for each stage state from the initial stage of the pothole that may occur in the road pavement to the occurrence, and since the pothole occurrence section of a road pavement can be easily identified and proactive maintenance is possible so that it contributes to ensuring the stability of passengers and roads.

According to the present invention, non-destructive and simple detection is possible while minimizing errors for each stage state from the initial stage of the pothole that may occur in the road pavement to the occurrence, and since the pothole occurrence section of a road pavement can be easily identified and proactive maintenance is possible so that it contributes to ensuring the stability of passengers and roads.

In addition, more accurate detection of pothole sign is possible by reflecting various environmental factors that can change the dielectric constant of a road pavement measured by electromagnetic wave.

In addition, more accurate detection of pothole sign is possible by reflecting various environmental factors that can change the attenuation of electromagnetic wave irradiated on a road pavement.

Although the present invention is described by some preferred embodiments, the scope of the present invention should not be limited thereby, but by the appended claims.

**Claims**

1. A pothole sign detection system (100) comprising:

   an electromagnetic wave transmitting unit (110) configured to emit electromagnetic waves, wherein the electromagnetic waves are tuned to penetrate through a surface of a road pavement and a plurality of pavement layer interfaces and to generate reflected waves from said surface of a road pavement and said plurality of pavement layer interfaces, respectively;
   an electromagnetic wave receiving unit (120) configured to receive the electromagnetic waves reflected from the surface of a road pavement and the plurality of pavement layer interfaces, respectively;
   a dielectric constant calculation unit (130) configured to calculate a dielectric constant of the surface of the road pavement and a dielectric constant of the pavement layer interface according to an amplitude of the received electromagnetic wave;
   a crack rate diagnosis unit (140) configured to diagnose a crack rate state of the surface of the road pavement by comparing the dielectric constant of the surface of the road pavement with a preset surface dielectric constant reference value;
   a pavement layer separation state diagnosis unit (150) configured to diagnose a pavement layer separation state of the pavement layer interface of the road pavement by comparing the dielectric constant of the pavement layer interface of the road pavement with a preset interface dielectric constant reference value;
   an attenuation calculation unit (160) configured to calculate energy attenuation in each of the plurality of pavement layers according to the amplitude of the received electromagnetic wave;
   a pavement layer inside state diagnosis unit (170) configured to diagnose a state inside the plurality of pavement layers by comparing the attenuation with a preset attenuation reference value; and
   a sign determination unit (180) configured to determine a pothole occurrence sign in the road pavement by using the crack rate state information, the pavement layer separation state information, and the attenuation information.

2. The pothole sign detection system (100) of claim 1, wherein the dielectric constant of the road pavement surface is calculated by Equation

$$\sqrt{\epsilon_{r0}} = \frac{1 + Amp_0}{1 - \dfrac{Amp_0}{Amp_P}}.$$

   where $\epsilon_{r0}$ is a dielectric constant of the road surface, $Amp_0$ is an amplitude of the electromagnetic wave at the road surface, and $Amp_P$ is an amplitude of the electromagnetic wave at a steel plate.

3. The pothole sign detection system (100) of claim 1 or 2, wherein the dielectric constant of the pavement layer interface is calculated by Equation

$$\sqrt{\epsilon_{rn}} = \sqrt{\epsilon_{r(n-1)}} \times \left[ \frac{1 - \left(\frac{Amp_{(n-1)}}{Amp_p}\right)^2 + \left(\frac{Amp_n}{Amp_p}\right)}{1 - \left(\frac{Amp_{(n-1)}}{Amp_p}\right)^2 - \left(\frac{Amp_n}{Amp_p}\right)} \right],$$

where $\epsilon_{rn}$ is a dielectric constant of the n-th pavement interface of the pavement road, and $Amp_n$ is an amplitude of the electromagnetic wave at the n-th pavement interface of the road pavement.

4. The pothole sign detection system (100) of any of claim 1 to 3, wherein the attenuation is calculated by Equation

$$\delta_n(dB) = 20\log_{10}\frac{Amp_n}{Amp_p} - 20\log_{10}\frac{Amp_{(n-1)}}{Amp_p}.$$

where $\delta_n$ is an attenuation (dB) of the electromagnetic wave passing through the n-th pavement layer of the road pavement.

5. The pothole sign detection system (100) of any of claim 1 to 4, further comprising a reference value correction unit (190) configured to correct the surface dielectric constant reference value and the interface dielectric constant reference value according to a factor in the road pavement.

6. The pothole sign detection system (100) of claim 5, wherein the factor is age and relative humidity, wherein the dielectric constant reference value is calculated by Equation $\epsilon_r = A \cdot W_r - B\ln(Age) + C$, where $\epsilon_r$ is the dielectric constant reference value, $W_r$ is a water content or relative humidity (%), Age is the material age of concrete or years of public use, and A, B, C are predetermined constants.

7. The pothole sign detection system (100) of claim 6, wherein when the pavement layer is asphalt, B is set to 0, and A and C are differently set for the surface of the road pavement and the internal pavement interface, wherein A is set to be smaller by a predetermined ratio as compared to the surface of the road pavement for the internal pavement interface of the road pavement.

8. The pothole sign detection system (100) of claim 6, wherein when the pavement layer is concrete, A, B, and C are differently set for the surface of the road pavement and the internal pavement interface, respectively, wherein B is set to be smaller than the surface of the road pavement for the internal pavement interface of the road pavement.

9. The pothole sign detection system (100) of claim 5, wherein the factor is a density of a pavement layer of the road pavement, wherein the dielectric constant reference value is calculated by Equation $\epsilon_r = a\rho + b$, where $\epsilon_r$ is the dielectric constant reference value, $\rho$ is the density of a core specimen of the road pavement, and a and b are constants.

10. The pothole sign detection system (100) of claim 5, wherein the reference value correction unit (190) further corrects an attenuation reference value according to a factor in the road pavement.

11. The pothole sign detection system (100) of claim 10, wherein the factor is a density of the pavement layer of the road pavement, wherein the attenuation reference value is calculated by Equation $\sigma = a\rho - b$, where $\sigma$ is the attenuation reference value, $\rho$ is the density of a core specimen of the road pavement, and a and b are constants.

12. A pothole sign detection method performed by the pothole sign detection system (100) in any of claims 1 to 11, the method comprising:

   emitting, by an electromagnetic wave transmitting unit (110), electromagnetic waves;

receiving, by an electromagnetic wave receiving unit (120), electromagnetic waves reflected from a surface of a road pavement and a plurality of pavement layer interfaces, respectively;

calculating, by a dielectric constant calculation unit (130), a dielectric constant of the surface of the road pavement and the pavement layer interface according to an amplitude of the received electromagnetic wave;

diagnosing, by a crack rate diagnosis unit (140), a crack rate state of the surface of the road pavement by comparing a dielectric constant of the surface of the road pavement with a preset surface dielectric constant reference value;

diagnosing, by a pavement layer separation state diagnosis unit (150), a pavement layer separation state of the pavement layer interface of the road pavement by comparing a dielectric constant of the pavement layer interface of the road pavement with a preset interface dielectric constant reference value;

calculating, by an attenuation calculation unit (160), energy attenuation in each of the plurality of pavement layers according to the amplitude of the received electromagnetic wave;

diagnosing, by a pavement layer inside state diagnosis unit (170), a state inside the plurality of pavement layers by comparing the attenuation with a preset attenuation reference value; and

determining, by a sign determination unit (180), a pothole occurrence sign in the road pavement by using the crack rate state information, the pavement layer separation state information, and the attenuation information.

## Patentansprüche

1. Schlaglochanzeichen-Detektionssystem (100), umfassend:

eine Übertragungseinheit (110) elektromagnetischer Wellen, welche dazu eingerichtet ist, elektromagnetische Wellen zu emittieren, wobei die elektromagnetischen Wellen abgestimmt sind, um eine Oberfläche eines Straßenbelags und eine Mehrzahl von Belagschicht-Grenzflächen zu durchdringen und von der Oberfläche eines Straßenbelags bzw. der Mehrzahl von Belagschicht-Grenzflächen reflektierte Wellen zu erzeugen;

eine Empfangseinheit (120) elektromagnetischer Wellen, welche dazu eingerichtet ist, die von der Oberfläche eines Straßenbelags bzw. der Mehrzahl von Belagschicht-Grenzflächen reflektierten elektromagnetischen Wellen zu empfangen;

eine Berechnungseinheit (130) einer dielektrischen Konstante, welche dazu eingerichtet ist, gemäß einer Amplitude der empfangenen elektromagnetischen Welle eine dielektrische Konstante der Oberfläche des Straßenbelags und eine dielektrische Konstante der Belagschicht-Grenzfläche zu berechnen;

eine Fehlstellenrate-Diagnoseeinheit (140), welche dazu eingerichtet ist, einen Fehlstellenrate-Zustand der Oberfläche des Straßenbelags durch Vergleichen der dielektrischen Konstante der Oberfläche des Straßenbelags mit einem voreingestellten Referenzwert einer dielektrischen Konstante einer Oberfläche zu diagnostizieren;

eine Diagnoseeinheit (150) für einen Straßenbelag-Trennungszustand, welche dazu eingerichtet ist, einen Straßenbelag-Trennungszustand der Belagschicht-Grenzfläche des Straßenbelags durch Vergleichen der dielektrischen Konstante der Belagschicht-Grenzfläche des Straßenbelags mit einem voreingestellten Referenzwert einer dielektrischen Konstante einer Grenzfläche zu diagnostizieren;

eine Dämpfungsberechnungseinheit (160), welche dazu eingerichtet ist, gemäß der Amplitude der empfangenen elektromagnetischen Welle eine Energiedämpfung in jeder der Mehrzahl von Belagschichten zu berechnen;

eine Diagnoseeinheit (170) für einen inneren Zustand einer Belagschicht, welche dazu eingerichtet ist, durch Vergleichen der Dämpfung mit einem voreingestellten Dämpfungsreferenzwert einen Zustand innerhalb der Mehrzahl von Belagschichten zu diagnostizieren; und

eine Anzeichenbestimmungseinheit (180), welche dazu eingerichtet ist, unter Verwendung der Fehlstellenrate-Zustandsinformationen, der Straßenbelag-Trennungszustandsinformationen und der Dämpfungsinformationen ein Anzeichen für ein Auftreten eines Schlaglochs in dem Straßenbelag zu bestimmen.

2. Schlaglochanzeichen-Detektionssystem (100) nach Anspruch 1, wobei die dielektrische Konstante der Straßenbelagoberfläche durch eine Gleichung

$$\sqrt{\epsilon_{r0}} = \frac{1 + Amp_0}{1 - \dfrac{Amp_0}{Amp_P}},$$

berechnet ist,
wobei $\epsilon_{r0}$ eine dielektrische Konstante der Straßenoberfläche ist, $Amp_0$ eine Amplitude der elektromagnetischen Welle an der Straßenoberfläche ist, und $Amp_P$ eine Amplitude der elektromagnetischen Welle an einer Stahlplatte ist.

3. Schlaglochanzeichen-Detektionssystem (100) nach Anspruch 1 oder 2, wobei die dielektrische Konstante der Belagschicht-Grenzfläche durch eine Gleichung

$$\sqrt{\epsilon_{rn}} = \sqrt{\epsilon_{r(n-1)}} \times \left[ \frac{1 - \left(\frac{Amp_{(n-1)}}{Amp_{\hat{p}}}\right)^2 + \left(\frac{Amp_n}{Amp_p}\right)}{1 - \left(\frac{Amp_{(n-1)}}{Amp_p}\right)^2 - \left(\frac{Amp_n}{Amp_p}\right)} \right],$$

berechnet ist,
wobei $\epsilon_{rn}$ eine dielektrische Konstante an der n-ten Belag-Grenzfläche des Straßenbelags ist, und $Amp_n$ eine Amplitude der elektromagnetischen Welle an der n-ten Belag-Grenzfläche des Straßenbelags ist.

4. Schlaglochanzeichen-Detektionssystem (100) nach einem der Ansprüche 1 bis 3, wobei die Dämpfung durch eine Gleichung

$$\delta_n(dB) = 20\log_{10}\frac{Amp_n}{Amp_p} - 20\log_{10}\frac{Amp_{(n-1)}}{Amp_p},$$

berechnet ist,
wobei $\delta_n$ eine Dämpfung (dB) der elektromagnetischen Welle ist, welche durch die n-te Belagschicht des Straßenbelags verläuft.

5. Schlaglochanzeichen-Detektionssystem (100) nach einem der Ansprüche 1 bis 4, ferner umfassend eine Referenzwert-Korrektureinheit (190), welche dazu eingerichtet ist, den Referenzwert einer dielektrischen Konstante einer Oberfläche und den Referenzwert einer dielektrischen Konstante einer Grenzfläche gemäß einem Faktor in dem Straßenbelag zu korrigieren.

6. Schlaglochanzeichen-Detektionssystem (100) nach Anspruch 5, wobei der Faktor ein Alter und eine relative Luftfeuchtigkeit ist,
wobei der Referenzwert einer dielektrischen Konstante durch eine Gleichung $\epsilon_r = A \cdot W_r - B\ln(Age) + C$ berechnet ist, wobei $\epsilon_r$ der Referenzwert einer dielektrischen Konstante ist, $W_r$ ein Wassergehalt oder eine relative Luftfeuchtigkeit (%) ist, Age das Materialalter eines Betons oder Jahre öffentlicher Nutzung ist, und A, B, C vorbestimmte Konstanten sind.

7. Schlaglochanzeichen-Detektionssystem (100) nach Anspruch 6, wobei, wenn die Belagschicht Asphalt ist, B auf 0 eingestellt ist und A und C für die Oberfläche des Straßenbelags und die innere Belaggrenzfläche unterschiedlich eingestellt sind,
wobei A der Art eingestellt ist, dass es für die innere Belaggrenzfläche des Straßenbelags im Vergleich zu der Oberfläche des Straßenbelags um ein vorbestimmtes Verhältnis kleiner ist.

8. Schlaglochanzeichen-Detektionssystem (100) nach Anspruch 6, wobei, wenn die Belagschicht Beton ist, A, B und C für die Oberfläche des Straßenbelags bzw. die innere Belaggrenzfläche unterschiedlich eingestellt sind,
wobei B der Art eingestellt ist, dass es für die innere Belaggrenzfläche des Straßenbelags kleiner als die Oberfläche des Straßenbelags ist.

9. Schlaglochanzeichen-Detektionssystem (100) nach Anspruch 5, wobei der Faktor eine Dichte einer Belagschicht des Straßenbelags ist,
wobei der Referenzwert einer dielektrischen Konstante durch eine Gleichung $\epsilon_r = a\rho + b$, berechnet ist, wobei $\epsilon_r$ der Referenzwert einer dielektrischen Konstante ist, $\rho$ die Dichte einer Kernprobe des Straßenbelags ist, und a und b Konstanten sind.

10. Schlaglochanzeichen-Detektionssystem (100) nach Anspruch 5, wobei die Referenzwert-Korrektureinheit (190) ferner einen Dämpfungsreferenzwert gemäß einem Faktor in dem Straßenbelag korrigiert.

11. Schlaglochanzeichen-Detektionssystem (100) nach Anspruch 10, wobei der Faktor eine Dichte der Belagschicht des Straßenbelags ist,
wobei der Dämpfungsreferenzwert durch eine Gleichung $\sigma = a\rho - b$, berechnet ist, wobei $\sigma$ der Dämpfungsreferenzwert ist, $\rho$ die Dichte einer Kernprobe des Straßenbelags ist, und a und b Konstanten sind.

12. Schlaglochanzeichen-Detektionsverfahren, welches durch das Schlaglochanzeichen-Detektionssystem (100) in einem der Ansprüche 1 bis 11 durchgeführt wird, wobei das Verfahren umfasst:

Emittieren, durch eine Übertragungseinheit (110) elektromagnetischer Wellen, elektromagnetischer Wellen;
Empfangen, durch eine Empfangseinheit (120) elektromagnetischer Wellen, elektromagnetischer Wellen, die von einer Oberfläche eines Straßenbelags bzw. einer Mehrzahl von Belagschicht-Grenzflächen reflektiert werden;
Berechnen, durch eine Berechnungseinheit (130) einer dielektrischen Konstante, einer dielektrischen Konstante der Oberfläche des Straßenbelags und der Belagschicht-Grenzfläche gemäß einer Amplitude der empfangenen elektromagnetischen Welle;
Diagnostizieren, durch eine Fehlstellenrate-Diagnoseeinheit (140), eines Fehlstellenrate-Zustands der Oberfläche des Straßenbelags durch Vergleichen einer dielektrischen Konstante der Oberfläche des Straßenbelags mit einem voreingestellten Referenzwert einer dielektrischen Konstante einer Oberfläche;
Diagnostizieren, durch eine Diagnoseeinheit (150) für einen Straßenbelag-Trennungszustand, eines Straßenbelag-Trennungszustands der Belagschicht-Grenzfläche des Straßenbelags durch Vergleichen der dielektrischen Konstante der Belagschicht-Grenzfläche des Straßenbelags mit einem voreingestellten Referenzwert einer dielektrischen Konstante einer Grenzfläche;
Berechnen, durch eine Dämpfungsberechnungseinheit (160), einer Energiedämpfung in jeder der Mehrzahl von Belagschichten gemäß der Amplitude der empfangenen elektromagnetischen Welle;
Diagnostizieren, durch eine Diagnoseeinheit (170) für einen inneren Zustand einer Belagschicht, eines Zustands innerhalb der Mehrzahl von Belagschichten durch Vergleichen der Dämpfung mit einem voreingestellten Dämpfungsreferenzwert; und
Bestimmen, durch eine Anzeichenbestimmungseinheit (180), eines Anzeichens für ein Auftreten eines Schlaglochs in dem Straßenbelag, unter Verwendung der Fehlstellenrate-Zustandsinformationen, der Straßenbelag-Trennungszustandsinformationen und der Dämpfungsinformationen.

**Revendications**

1. Un système de détection de signe de nid-de-poule (100) comprenant :

une unité de transmission d'ondes électromagnétiques (110) configurée pour émettre des ondes électromagnétiques, les ondes électromagnétiques étant réglées de façon à pénétrer à travers une surface d'un revêtement routier et une pluralité d'interfaces de couche de revêtement et de façon à générer des ondes réfléchies depuis ladite surface d'un revêtement routier et ladite pluralité d'interfaces de couche de revêtement, respectivement ;
une unité de réception d'ondes électromagnétiques (120) configurée pour recevoir les ondes électromagnétiques réfléchies par la surface d'un revêtement routier et la pluralité d'interfaces de couche de revêtement, respectivement ;
une unité de calcul de constante diélectrique (130) configurée pour calculer une constante diélectrique de la surface du revêtement routier et une constante diélectrique de l'interface de couche de revêtement en fonction d'une amplitude de l'onde électromagnétique reçue ;
une unité de diagnostic de taux de fissurations (140) configurée pour diagnostiquer un état de taux de fissurations de la surface du revêtement routier en comparant la constante diélectrique de la surface du revêtement routier avec une valeur de référence prédéfinie de constante diélectrique de surface ;
une unité de diagnostic d'état de séparation de couche de revêtement (150) configurée pour diagnostiquer un état de séparation de couche de revêtement de l'interface de couche de revêtement du revêtement routier en comparant la constante diélectrique de l'interface de couche de revêtement du revêtement routier avec une valeur de référence de constante diélectrique d'interface prédéfinie ;

une unité de calcul d'atténuation (160) configurée pour calculer l'atténuation d'énergie dans chacune de la pluralité de couches de revêtement en fonction de l'amplitude de l'onde électromagnétique reçue ;

une unité de diagnostic d'état intérieur de couche de revêtement (170) configurée pour diagnostiquer un état à l'intérieur de la pluralité de couches de revêtement en comparant l'atténuation avec une valeur de référence de l'atténuation prédéfinie ; et

une unité de détermination de signe (180) configurée pour déterminer un signe d'occurrence de nid-de-poule dans le revêtement routier en utilisant les informations d'état de taux de fissuration, les informations d'état de séparation de couche de revêtement et les informations d'atténuation.

2. Le système de détection de signe de nid-de-poule (100) selon la revendication 1, dans lequel la constante diélectrique de la surface du revêtement est calculée par l'équation

$$\sqrt{\epsilon_{r0}} = \frac{1 + Amp_0}{1 - \dfrac{Amp_0}{Amp_P}}$$

dans laquelle $\epsilon_{r0}$ est une constante diélectrique de la surface de la route, $Amp_0$ est une amplitude de l'onde électromagnétique à la surface de la route et $Amp_P$ est une amplitude de l'onde électromagnétique au niveau d'une plaque d'acier.

3. Le système de détection de signe de nid-de-poule (100) selon la revendication 1 ou la revendication 2, dans lequel la constante diélectrique de l'interface de couche de revêtement est calculée par l'équation

$$\sqrt{\epsilon_{rn}} = \sqrt{\epsilon_{r(n-1)}} \times \left[ \frac{1 - \left(\dfrac{Amp_{(n-1)}}{Amp_p}\right)^2 + \left(\dfrac{Amp_n}{Amp_p}\right)}{1 - \left(\dfrac{Amp_{(n-1)}}{Amp_p}\right)^2 - \left(\dfrac{Amp_n}{Amp_p}\right)} \right],$$

dans laquelle $\epsilon_m$ est une constante diélectrique de la n-ième interface de revêtement du revêtement routier, et $Amp_n$ est une amplitude de l'onde électromagnétique à la n-ième interface de revêtement du revêtement routier.

4. Le système de détection de signe de nid-de-poule (100) selon l'une quelconque des revendications 1 à 3, dans lequel l'atténuation est calculée par l'équation

$$\delta_n(dB) = 20\log_{10}\frac{Amp_n}{Amp_p} - 20\log_{10}\frac{Amp_{(n-1)}}{Amp_p}$$

dans laquelle $\delta_n$ est une atténuation (dB) de l'onde électromagnétique traversant la n-ième couche de revêtement du revêtement routier.

5. Le système de détection de signe de nid-de-poule (100) selon l'une quelconque des revendications 1 à 4, comprenant en outre une unité de correction de valeur de référence (190) configurée pour corriger la valeur de référence de constante diélectrique de surface et la valeur de référence de constante diélectrique d'interface en fonction d'un facteur dans la chaussée de la route.

6. Le système de détection de signe de nid-de-poule (100) selon la revendication 5, dans lequel le facteur est l'âge et l'humidité relative,
la valeur de référence de la constante diélectrique étant calculée par l'équation $\epsilon_r = A \cdot W_r - B\ln(Age) + C$, dans laquelle $\epsilon_r$ est la valeur de référence de la constante diélectrique, $W_r$ est une teneur en eau ou une humidité relative (%), Age est l'âge du matériau de béton ou des années d'utilisation publique, et A, B, C sont des constantes prédéterminées.

7. Le système de détection de signe de nid-de-poule (100) selon la revendication 6, dans lequel lorsque la couche de revêtement est de l'asphalte, B est définie à 0, et A et C sont définies différemment pour la surface du revêtement routier et l'interface de revêtement interne,
A étant définie de façon à être plus petite d'un rapport prédéterminé par comparaison avec la surface du revêtement routier pour l'interface de revêtement interne du revêtement routier.

8. Le système de détection de signe de nid-de-poule (100) selon la revendication 6, dans lequel lorsque la couche de revêtement est en béton, A, B et C sont définies différemment pour la surface du revêtement routier et l'interface de revêtement interne, respectivement,
B étant définie de façon à être plus petite que la surface du revêtement routier pour l'interface de revêtement interne du revêtement routier.

9. Le système de détection de signe de nid-de-poule (100) selon la revendication 5, dans lequel le facteur est une densité d'une couche de revêtement du revêtement routier,
la valeur de référence de la constante diélectrique étant calculée par l'équation $c_r = a\rho + b$, dans laquelle $\epsilon_r$ est la valeur de référence de la constante diélectrique, $\rho$ est la densité d'une carotte du revêtement routier, et a et b sont des constantes.

10. Le système de détection de signe de nid-de-poule (100) selon la revendication 5, dans lequel l'unité de correction de valeur de référence (190) corrige en outre une valeur de référence de l'atténuation en fonction d'un facteur dans le revêtement routier.

11. Le système de détection de signe de nid-de-poule (100) selon la revendication 10, dans lequel le facteur est une densité de la couche de revêtement du revêtement routier,
la valeur de référence de l'atténuation étant calculée par l'équation $\sigma = a\rho - b$, dans laquelle $\sigma$ est la valeur de référence de l'atténuation, $\rho$ est la densité d'une carotte du revêtement routier, et a et b sont des constantes.

12. Un procédé de détection de signe de nid-de-poule mis en œuvre par le système de détection de signe de nid-de-poule (100) selon l'une quelconque des revendications 1 à 11, le procédé comprenant :

le fait d'émettre, par une unité de transmission d'ondes électromagnétiques (110), des ondes électromagnétiques ;
le fait de recevoir, par une unité de réception d'ondes électromagnétiques (120), des ondes électromagnétiques réfléchies par une surface d'un revêtement routier et une pluralité d'interfaces de couche de revêtement, respectivement ;
le fait de calculer, par une unité de calcul de constante diélectrique (130), une constante diélectrique de la surface du revêtement routier et de l'interface de couche de revêtement en fonction d'une amplitude de l'onde électromagnétique reçue ;
le fait de diagnostiquer, par une unité de diagnostic de taux de fissurations (140), un état de taux de fissuration de la surface du revêtement routier en comparant une constante diélectrique de la surface du revêtement routier à une valeur de référence prédéfinie de constante diélectrique de surface ;
le fait de diagnostiquer, par une unité de diagnostic d'état de séparation de couche de revêtement (150), un état de séparation de couche de revêtement de l'interface de couche de revêtement du revêtement routier en comparant une constante diélectrique de l'interface de couche de revêtement du revêtement routier avec une valeur de référence prédéfinie de constante diélectrique d'interface ;
le fait de calculer, par une unité de calcul d'atténuation (160), l'atténuation énergétique dans chaque couche de la pluralité de couches de revêtement en fonction de l'amplitude de l'onde électromagnétique reçue ;
le fait de diagnostiquer, par une unité de diagnostic d'état intérieur de couche de revêtement (170), un état à l'intérieur de la pluralité de couches de revêtement en comparant l'atténuation à une valeur de référence de l'atténuation prédéfinie ; et
le fait de déterminer, par une unité de détermination de signe (180), un signe d'occurrence de nid-de-poule dans le revêtement routier en utilisant les informations d'état de taux de fissuration, les informations d'état de séparation de couche de revêtement et les informations d'atténuation.

100

## POTHOLE SIGN DETECTION SYSTEM

| | |
|---|---|
| ELECTROMAGNETIC WAVE TRANSMITTING UNIT | ~110 |
| ELECTROMAGNETIC WAVE RECEIVING UNIT | ~120 |
| DIELECTRIC CONSTANT CALCULATION UNIT | ~130 |
| CRACK RATE DIAGNOSIS UNIT | ~140 |
| PAVEMENT LAYER SEPARATION STATE DIAGNOSIS UNIT | ~150 |
| ATTENUATION CALCULATION UNIT | ~160 |
| PAVEMENT LAYER INSIDE STATE DIAGNOSIS UNIT | ~170 |
| SIGN DETERMINATION UNIT | ~180 |
| REFERENCE VALUE CORRECTION UNIT | ~190 |

# FIG. 1

FIG. 2

PAVEMENT LAYER THICKNESS AND INTERNAL INFORMATION (SEPARATE NONDESTRUCTIVE INSPECTION, CORE, CUTTING INSPECTION, ETC.)

PAVEMENT ATTACHMENT INFORMATION (HITTING INSPECTION, CORE, LFWD, ETC.)

WATER CONTENT INFORMATION (ATMOSPHERIC RELATIVE HUMIDITY, WEATHER, CORE WEIGHT, ETC.)

CRACK RATE INFORMATION (DESIGN POROSITY, ROAD SURFACE IMAGE, ETC.)

ROAD INFORMATION (MATERIAL AGE, YEARS OF PUBLIC USE, LOCATION, ETC.)

DATA RECEIVING UNIT

DATA PREPROCESSING UNIT

DIELECTRIC CONSTANT CALCULATION UNIT

ATTENUATION CALCULATION UNIT

CONTROL UNIT

ELECTROMAGNETIC WAVE GENERATION UNIT

ELECTROMAGNETIC WAVE TRANSMITTING/RECEIVING UNIT

DATA EXTRACTION UNIT

DATA STORAGE UNIT

PORTHOLE SIGN DETERMINATION UNIT
(1) DATA CORRECTION : HUMIDITY, WEATHER, AGE, THICKNESS
(2) DATA COMPARISON (CRACK RATE, WATER CONTENT RATIO, ADHESION, INTERNAL STATE)
(3) POTHOLE SIGN DETERMINATION

19

FIG. 3

$$\epsilon = a\rho + b$$

FIG. 4

FIG. 5

FIG. 6

START

INPUT ELECTROMAGNETIC DATA — S110

CALCULATE DIELECTRIC CONSTANT AND ATTENUATION VALUE
·CALCULATE PAVEMENT LAYER UPPER
  SURFACE DIELECTRIC CONSTANT
·CALCULATE PAVEMENT LAYER LOWER
  SURFACE DIELECTRIC CONSTANT
·CALCULATE PAVEMENT LAYER ATTENUATION AMOUNT — S120

PAVEMENT LAYER LOWER SURFACE WITHIN THRESHOLD VALUE (RANGE)? — no → EXPRESS 'POTHOLE SIGN' — S130

yes

LESS THAN PAVEMENT LAYER ATTENUATION THRESHOLD VALUE? — no → EXPRESS 'POTHOLE SIGN' — S140

yes

PAVEMENT LAYER UPPER SURFACE WITHIN THRESHOLD VALUE (RANGE)? — no → EXPRESS 'POTHOLE BEGINNING' — S150

yes

EXPRESS 'GOOD' — S160

END

FIG. 7

**REFERENCES CITED IN THE DESCRIPTION**

**Patent documents cited in the description**

- KR 101761165
- KR 101635323
- KR 101514368
- KR 101546700
- KR 101675928
- KR 101537247
- KR 20190024416 A
- US 2019094202 A1
- JP 2010014472 A